# EUROPEAN PATENT APPLICATION

(11) **EP 4 586 266 A1**
(43) Date of publication of application: **16.07.2025**
(21) Application number: 24151862.0
(22) Date of filing: 15.01.2024
(51) Int. Cl.: G16H 30/40, G16H 40/63, G16H 50/20, G16H 50/70

(54) **MEDICAL IMAGE VISUALIZATION**

(71) Applicant: Koninklijke Philips N.V., 5656 AG Eindhoven (NL)
(72) Inventor: LAVES, Max-Heinrich Viktor, Eindhoven (NL); LOSSAU, Tanja, Eindhoven (NL); BUERGER, Christian, 5656AG Eindhoven (NL); LORENZ, Cristian, Eindhoven (NL); GOLLA, Alena-Kathrin, Eindhoven (NL); LIN, Qizhong, Eindhoven (NL); CHEN, Hongxin, Eindhoven (NL); KLINDER, Tobias, Eindhoven (NL)
(74) Representative: Philips Intellectual Property & Standards

(57) **Abstract**

A method and system for configuring a user interface of a medical image visualization system based on an AI anatomical or pathology classification of an image being displayed. In particular, the system response to a given user input command may be automatically adapted in dependence upon an anatomy being displayed. For example, an image rendering setting used to render a new image view responsive to a user command may change depending on the anatomy. For example, a change in a zoom setting applied in rendering a new image view responsive to a user input command may change depending on the anatomy.

## Description

### FIELD OF THE INVENTION

This invention relates to the field of medical image visualization, and to a user interface for use in medical image visualization.

### BACKGROUND OF THE INVENTION

Medical images are reviewed by radiologists to identify potential pathologies. During a review session, much time is spent adjusting settings of the user interface being used to view the images to obtain the optimal view and/or the optimal mode of the user interface device. For example, much time may be spent zooming in and out between local and global views and adjusting various other settings of the visualization of the data to assess subtle findings efficiently. This may be particularly the case when reviewing medical images for polytraumatic patients for which review is required for multiple different local sites of an imaged anatomy.

This excess time spent configuring settings of the user interface can negatively affect the chance of survival of the patient due to delayed information necessary for the appropriate treatment. Additionally, tedious navigation and inconvenient user interfaces can lead to missed and overlooked pathologies, such as rib or vertebral fractures. For that reason, a high number of rib fractures can be missed even by trained radiologists. This underlines the importance of more elaborate tools for navigating dense three-dimensional image data.

For example, one application is review of computed tomography (CT) images. For example, one particular application is the review of chest CT images for polytraumatic patients. CT is the recommended imaging modality for major body trauma, including fractures of the ribs. Currently, techniques such as volume rendering of full-body acquisitions and/or multiplanar reformation (MPR) are used to visualize acquired image data and assist the radiologist in finding polytraumas. When reading such a polytraumatic scan, much reading time may often be spent zooming in and out between local and global views and adjusting the visualization of the data to assess subtle findings efficiently.

### SUMMARY OF THE INVENTION

It is the recognition of the inventors that time spent configuring the user interface of a medical image visualization system may be reduced by improving the user input mechanism. The range of image rendering options for displaying the image may in many cases be adequate, but the means for controlling their selection is often slow, inefficient, and inconvenient. Thus, it is the recognition of the inventors that an improved user input means for a medial image display visualization system would be of benefit.

The invention is defined by the claims.

According to examples in accordance with an aspect of the invention, there is provided a method of configuring user input of a medical image visualization system.

The method comprises displaying on a display device of a user interface a first image rendering of a medical image dataset. The method further comprises receiving from a user input device of the user interface a user input command. The method further comprises applying to the medical image dataset an AI classification model configured to output classification data pertaining to at least a region of the image data. The AI classification model may be an AI anatomical classification model configured to output anatomical classification data pertaining to at least a region of the image data. Additionally or alternatively, the AI classification model may be an AI pathology classification model configured to output pathological classification data pertaining to at least a region of the image data. The method further comprises performing a system operation on the visualization system responsive to the user input command. The method further comprises selecting or configuring the system operation which is performed responsive to the received user input command in dependence upon the classification data, for example based on a pre-defined command translation protocol.

It is thus proposed to provide a user input mechanism which intelligently and automatically reconfigures a mapping between user input commands and a resultant system operation in dependence upon properties of the anatomy currently being displayed. Thus, a user is able to control the settings of the visualization system using only a limited set of input commands, and wherein the system automatically infers the correct change to the visualization system, e.g. changing a rendered image view, in dependence upon an output from the classification module.

For example, at least some embodiments of the invention aim to address the problem of automatically finding the right image viewport configuration for a given displayed anatomy and/or pathology, and therefore potentially reducing reading times considerably. Embodiments may positively affect both the treatment of the patient and the satisfaction of the physician in using products equipped with this invention. Moreover, it can readily be applied to a variety of medical image modalities, including CT, MRI, ultrasound, and X-ray.

With regards to the received user input command, in some embodiments, this could be a command which is indicative of a location on the first image rendering being displayed. For example, the received user input command might be a pointer click or double click or other gesture at a location on the displayed first image rendering.

In some embodiments, the AI classification model may be configured to output classification data pertaining to a plurality of segmented image regions within the image dataset. The selecting or configuring system operation may comprise selecting or configuring the system operation based on the classification in the output classification data assigned to the image region containing the user-indicated location on the image. For instance, based on anatomical or pathology classification information generated by the classification model, a resultant system command may be triggered, for instance related to the anatomy area or pathology indicated by the input command.

In some embodiments, the system operation may comprise a processing operation applied to the image dataset. For example, in some embodiments, the system operation may comprise an image rendering operation applied to the image dataset.

In some embodiments, the system operation comprises an image rendering operation applied to the image dataset to generate a second image rendering of the image dataset and outputting the second image rendering to the display device for display.

In some embodiments, adjusting the system operation comprises adjusting rendering parameters of the image rendering operation applied to the image dataset to generate the second image rendering. The rendering parameters of the image rendering operation may include a zoom setting of the image, for example a zoom level and/or a zoom center point, or visualization contrast by setting intensity level/window values.

In some embodiments, the first image rendering of the image dataset may represent a global view of the image dataset. In some embodiments, the second image rendering of the image dataset may represent a local view of the image dataset, wherein the local view has a greater zoom level than the global view. The area covered by the local point may be determined based on the user input command, e.g. based on a location in the first image selected by the user as part of the user input command.

In some embodiments, following display of the second image rendering, the method comprises receiving from the user input device of the user interface a second user input command, the same as or different to the first user input command, and wherein, responsive to said receipt, the method comprises switching the display device to display of the first image rendering. In other words, the user is enabled to switch between the first and second image renderings, for example first and second rendered views of the anatomy, using user input commands. For example, the user may be enabled to toggle between display of the first and second image renderings.

In some embodiments, the user interface comprises a user input mechanism comprising the user input device and a command translator. The performing the system operation responsive to the user input command may comprise the command translator translating the user input command to an assigned system operation command.

In some embodiments, the selecting or configuring the system operation which is performed responsive to the received user input command may comprise selecting or configuring the system operation command assigned to the user input command.

In some embodiments, the command translator may be configured to translate any of a defined set of possible user input commands to respective assigned system operation commands. The assignments of the system operation commands to the user input commands may in some embodiments be defined by a mapping schema comprised by the command translator, and wherein the mapping schema is adjustable to vary the system operation commands assigned to the user input commands.

In some embodiments, the method comprises adjusting the mapping schema to select or configure the respective system operation commands assigned to each of said set of user input commands in dependence upon the output classification data.

The previously mentioned pre-defined command translation protocol may define how the mapping schema is to be adjusted in dependence upon the classification data.

In some embodiments, the pre-defined command translation protocol is user-selectable from a list of different pre-defined command translation protocols. For example each corresponds to a reading or visualization protocol.

A further aspect of the invention is a computer program product comprising computer program code configured, when run on a processor, to cause the processor to perform a method in accordance with any embodiment described in this disclosure or in accordance with any claim of this application.

Another aspect of the invention is a processing device for a medical image visualization system. The processing device comprises one or more processors, configured to: control a display device of a user interface to display a first image rendering of a medical image dataset; receive from a user input device of the user interface a user input command; apply to the medical image dataset an AI classification model configured to output classification data pertaining to at least a region of the image data; perform a system operation on the visualization system responsive to the user input command. The one or more processors are further configured to select or configuring the system operation which is performed responsive to the received user input command in dependence upon the classification data. This may for example be based on a pre-defined command translation protocol.

In some embodiments, the AI classification model is an AI anatomical classification model configured to output anatomical classification data pertaining to at least a region of the image data. In some embodiments, the AI pathology classification model is a pathology classification or detection model configured to output pathology classification data pertaining to at least a region of the image data.

Another aspect of the invention is an image visualization system comprising: a user interface comprising: a display device and a user input device; and a processing device as outlined above or in accordance with any embodiment described in this document or in accordance with any claim, operatively coupled to the user interface.

The user input device may comprise a user command detector adapted to detect user commands and generate command signals indicative thereof.

The user input device may comprise a command translator adapted to translate the user command signals to system command signals indicative of a system operation to be performed responsive to the user command signal, based on the pre-defined command translation protocol.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 is a flow diagram illustrating a basic processing flow according to one or more embodiments of the invention;
Fig. 4 is a flow diagram illustrating configuration of a command translator based on image classification data;
Fig. 5 is a flow diagram illustrating configuration of a command translator based on image classification data and in accordance with a selected protocol;
Fig. 6 is a flow diagram illustrating configuration of a command translator based on configuring a mapping schema of a command translator; and
Fig. 7 schematically illustrates an example embodiment in which a second image rendering is generated responsive to a user input command.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for configuring a user interface of a medical image visualization system based on an AI anatomical classification of an image being displayed. In particular, the system response to a given user input command may be automatically adapted in dependence upon an anatomy being displayed. For example, an image rendering setting used to render a new image view responsive to a user command may change depending on the anatomy. For example, a change in a zoom setting or image contrast applied in rendering a new image view responsive to a user input command may change depending on the anatomy.

In some embodiments, a system is provided enabling clinicians to select target regions using a simple gesture, e.g. double-clicking with a computer mouse or double-tapping on a touch screen. In some embodiments, the system may infer the intended anatomical structure and configure a zoom level of a newly displayed visualization to an anatomy-specific setting. The initial image view setting may be remembered and the system may in some embodiments permit return to the initial setting by a further gesture, which may comprise simply repeating the first gesture.

For example, in state of the art CT image visualization, acquired images can be viewed via a graphical user interface which is known as a 'viewport'. The viewport permits adjustment of a view setting of acquired CT image data. In state of the art systems, the viewport of a CT scan is modified by manual user inputs and displayed on a screen. The process of analyzing a whole scan requires frequent switching between different zoom levels (e.g. overview vs. close-up view) and/or other view settings, e.g. view direction in the case of volume rendering.

In one example set of embodiments, the user is provided control means permitting switching between anatomy-specific close-up zoom levels and overview zoom levels by a defined input command or gesture, such as double-clicking on a computer mouse or double-tapping with a finger on a touch screen. The zoomed-in levels may be provided by an anatomy-specific model and can be adjusted, e.g. between local and global zoom levels, by user preference. The zoomed-out levels may be set by the user prior to initiating the zooming-in. Additionally, the zoom may follow predefined, anatomy-specific reading protocols. The described image navigation and zooming method is not limited to computed tomography and can be used for any planar or volumetric medical imaging modality.

Some embodiments may include: a machine learning model zoo for detecting an anatomical structure in image data; an anatomical zoom level and viewport configuration database, e.g. in the form of a command translator, and a user interface permitting a user to view rendered images and capture user input commands.

However, the general principle of adapting a user interface response to user input commands in dependence upon an anatomy being displayed has more general applicability and could be applied advantageously, not just to adjusting zoom settings associated with user input commands, but also to adjusting any other system operation performed responsive to a user input command.

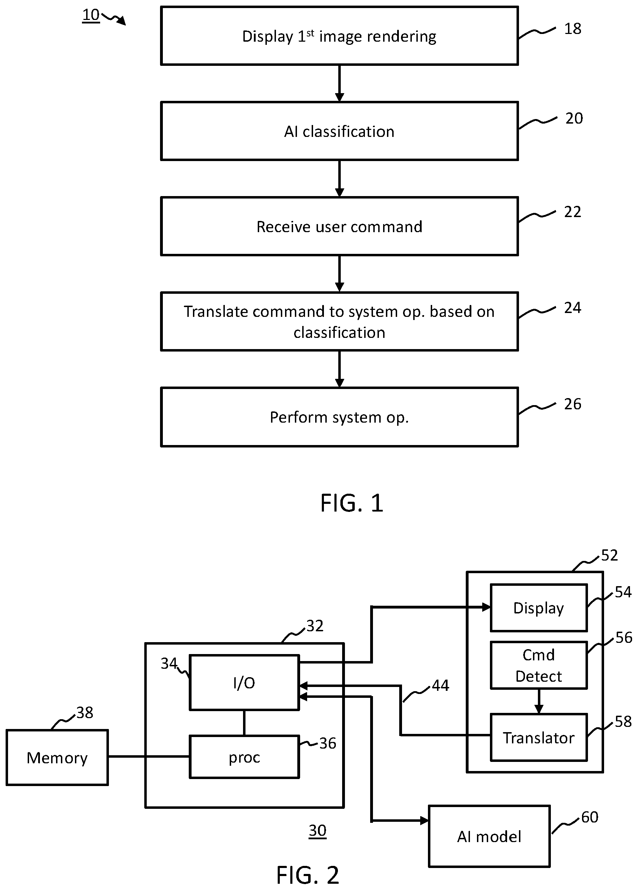

Fig. 1 outlines in block diagram form steps of an example method according to one or more embodiments. The steps will be recited in summary, before being explained further in the form of example embodiments.

Provided is a method 10 of configuring user input of a medical image visualization system. The method comprises displaying 18 on a display device of a user interface a first image rendering of a medical image dataset. The method further comprises applying 20 to the medical image dataset an AI classification model configured to output classification data pertaining to at least a region of the image data.

In some embodiments, the AI classification model may comprise an AI anatomical classification model configured to output anatomical classification data pertaining to at least a region of the image data. In some embodiments, additionally or alternatively, the AI classification model may comprise an AI pathology classification model configured to output pathology classification data pertaining to at least a region of the image data. For example, the pathology classification data may comprise detection of a pathology in at least said region of the image, e.g. a fracture. A pathology means for example an abnormality.

The method further comprises receiving 22 from a user input device of the user interface a user input command.

The method further comprises selecting or configuring 24 a system operation which is to be performed responsive to the received user input command in dependence upon the classification data, for example based on a pre-defined command translation protocol. This may be referred to as command translation. The method further comprises performing 26 the system operation in accordance with the selection or configuration on the visualization system responsive to the user input command.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52. The user interface may comprise a display device 54. The user interface may comprise a command detector 56 for detecting user input commands and generating user command signals indicative thereof. For example this may be coupled to or may comprise a user input device such as a pointer device or a touchscreen interface for capturing user input commands. The system may further comprise a command translator 58 adapted to translate the user command signals to system command signals 44 indicative of a system operation to be performed responsive to the user command signal, for example based on a pre-defined command translation protocol.

In the illustrated example of Fig. 2, the system 30 further comprises an AI classification model 60 for generating classification data 46 pertaining to at least a region of a set of input image data.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

With regards to the AI classification model 60, this may be a machine learning model. As mentioned above, the classification model may be an anatomy classification model and/or a pathology classification model.

A variety of suitable classification machine learning models exist in the field.

A machine learning anatomy classification model may be for anatomically segmenting a medical image. This therefore comprises localizing one or more different anatomical components of an imaged anatomy and tagging or labelling the localized anatomical components of the imaged anatomy. By way of non-limiting example, one suitable anatomy segmentation model is described in the following paper: Wasserthal, Jakob, et al. "TotalSegmentator: robust segmentation of 104 anatomical structures in CT images." arXiv preprint arXiv:2208.05868 (2022).

A machine learning pathology classification model may be for detecting one or more pathologies present in an input image. A wide variety of such models are known in the field. By way of just one example, the following paper describes a model for detecting rib fractures in images: Wu, Mingxiang, et al. "Development and evaluation of a deep learning algorithm for rib segmentation and fracture detection from multicenter chest CT images." Radiology: Artificial Intelligence 3.5 (2021).

In some embodiments, the system 30 may comprise an AI model datastore which stores one or more AI classification models, and wherein the method comprises retrieving a model from the datastore for application in performing the method. In some embodiments, the AI model datastore may store a plurality of different AI classification models. This may be referred to as a model zoo. The method 10 may comprise selecting one of the plurality of AI models for use in applying to the image dataset. The one of the models may be selected by a user via the user interface in some embodiments.

In general terms, the AI classification model may be configured to receive as input a medical image or a portion of a medical image and to generate as output anatomical and/or pathology classification data pertaining to at least a region of the image data. Anatomical classification data may comprise an identification of an anatomical structure or region or portion within the input image. may additionally or alternatively comprise a spatial isolation or detection of an area or volume covered by each of a set of one or more anatomical regions or structures, e.g. segmentation data. Pathology classification data may comprise detection of one or more pathologies for which the model is trained to detect within the input image. It additionally or alternatively may comprise a spatial isolation or detection of an area or volume covered by each of a set of one or more detected pathologies.

With regards to the user interface 52, this comprises a display device for displaying rendered image data. The user interface may use the display 54 to present a graphical user interface, where the graphical user interface may include a presentation of a rendered image rendered from the image data set. The user interface 52 also includes a user input means for capturing user input commands and translating these into system commands. The user input means may for example comprise a user input device for use by a user in inputting user commands, a command detector 56 for detector and/or capturing user input commands and a command translator 58 for translating or converting or mapping detected user inputs into responsive system commands 44.

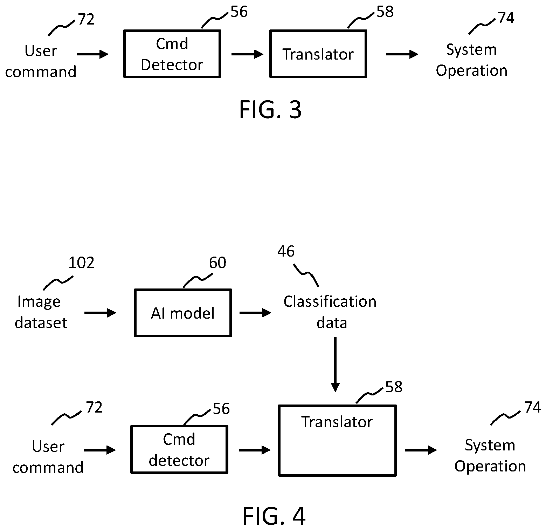

This is schematically illustrated in Fig. 3 which shows an example processing flow from a user command 72 to system operation 74. The user inputs a user command 72 via a user input device, a command detector 56 may detect or capture the user commands, and the command translator 58 may translate the captured user commands into a system operation 74.

The command translation depends on the classification data generated by the AI model. This is schematically illustrated by Fig. 4. As illustrated, it is proposed that the medical image dataset 102, or a portion thereof, from which the image rendering(s) are generated are provided as input to an AI classification model 60. The AI classification model generates anatomical and/or pathology classification data 46 pertaining to the input image dataset. This data is used to configure the command translator 58 so that the system operation 74 which is performed in response to a given user input command 72 captured by the command detector 56 may be modified in accordance with the anatomical and/or pathology classification data 46.

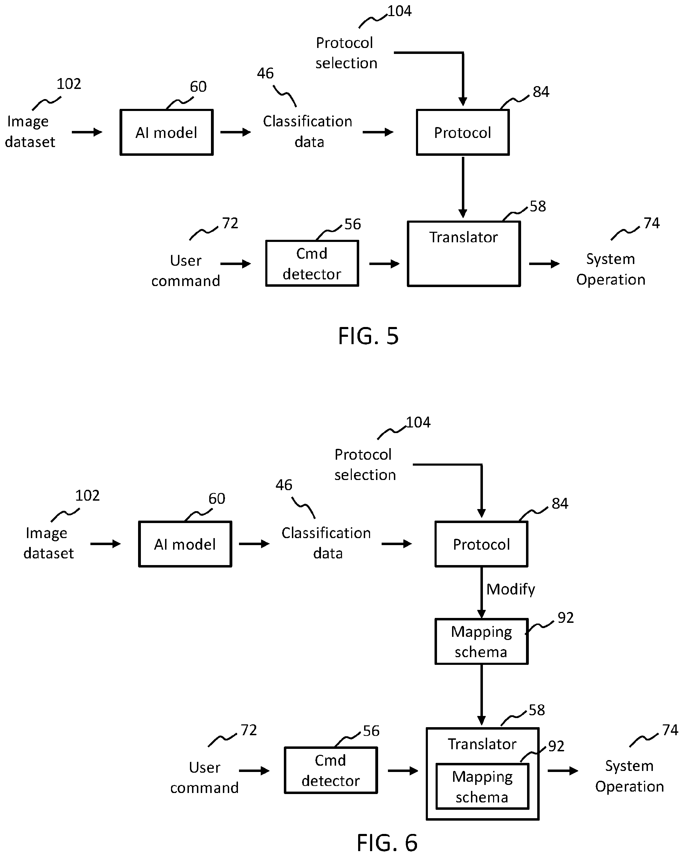

By way of example, the way in which the mapping between user commands 72 and system operations 74 is modified in dependence upon the classification data 46 may be determined by a defined command translation protocol 84. This is schematically illustrated in Fig. 5. The command translation protocol 84 may be selectable 104 in some embodiments, and may correspond to a particular viewing or visualization protocol or mode, or may correspond to a particular diagnostic protocol, or may relate to a particular pathology which is under investigation. The protocol may be user-selectable or may be selected automatically in some embodiments.

As schematically illustrated in Fig. 6, one way of implementing the configuration of the command translation in dependence upon the classification data 46 is to employ a configurable mapping schema 92 to define mappings between user commands 72 and system operations 74, and wherein the mapping schema 92 is adjusted, updated or replaced in dependence upon the classification data 46. For example, this modification of the mapping schema 92 could be determined or governed or defined by the command translation protocol 84 previously mentioned, which could optionally be selectable 104 as mentioned above.

With regards to the mapping schema 92, this provides one way of facilitating modification of system responses to a whole set of possible user input commands in dependence upon the anatomy or pathology being displayed. For example, the command translator 58 may be configured to translate any of a defined set of possible user input commands 72 to respective assigned system operation commands 74, and wherein the assignments of the system operation commands to the user input commands is defined by the mapping schema 92, which may in turn for example be comprised by the command translator 58. The mapping schema may then be adjustable to vary the system operation commands 74 assigned to the user input commands.

The method 10 may comprise adjusting the mapping schema 92 to select or configure the respective system operation commands 74 assigned to each of said set of user input commands 72 in dependence upon the output classification data 46.

The pre-defined command translation protocol 84 may define how the mapping schema 92 is to be adjusted in dependence upon the classification data 46.

With regards to the optional feature that the command translation protocol 84 be selectable 104 or configurable, in accordance with some embodiments, the pre-defined command translation protocol may be user-selectable from a list of different pre-defined command translation protocols. For example each may correspond to a reading or visualization protocol.

As briefly discussed above, one advantageous application for embodiments of the present invention is for use in providing adaptive visualization control when viewing medical images with a medical image visualization apparatus. In this context, a radiologist or other professional may need to switch frequently between different view settings or presets or defaults in order to diagnostically analyze the images. For example, it may be necessary to switch between different zoom settings, or different angular views in the case of volume rendering or multiplanar reforming (MPR) visualization of a volumetric dataset. Depending upon the anatomy and/or pathology represented in the image or in a portion of the image, different view settings may be relevant.

Changing a visualization view presented on the display 54 of an image dataset involves processing the image dataset with image rendering to render an image view. Depending upon the desired characteristics of the view to be visualized, rendering parameters of the rendering are adjusted.

Thus, the system operation(s) 74 executed responsive to the user input command(s) may comprise a processing operation applied to the image dataset 102. For example, as discussed above, the system operation may comprise an image rendering operation applied to the image dataset. The system operation 74 may comprise an image rendering operation applied to the image dataset 102 to generate a second image rendering of the image dataset and outputting the second image rendering to the display device 54 for display. The adjusting the system operation dependent upon the AI classification data 46 may then comprise adjusting rendering parameters of the image rendering operation applied to the image dataset to generate the second image rendering. In other words, the command translation by the command translator 58 may comprise translating the user commands 72 to particular image rendering operations by the system (i.e. rendering particular views of the image data), and the adjusting of the command translations, e.g. adjusting the mapping schema 92, may comprise changing rendering parameters of those rendering operations (i.e. changing the image views which are generated responsive to the same user input commands).

By way of example, rendering parameters of the image rendering operation may include a zoom setting of the image, for example a zoom level and/or a zoom center point. The zoom level may for example be defined relative to the overall image dataset. By way of further example, the rendering parameters may include a view direction of a volume rendered image relative to the image dataset or relative to an anatomical object or structure or pathology depicted in the image data. By way of further example, the rendering parameters may include a 2D plane cut across a 3D volumetric dataset for which an MPR rendering is to be generated.

As mentioned, the system operation 74 may comprise an image rendering operation applied to the image dataset 102 to generate a second image rendering of the image dataset and outputting the second image rendering to the display device 54 for display. In some embodiments, the first image rendering of the image dataset which is displayed on the display device 54 may represents a global view of the image dataset 102, and the second image rendering of the image dataset may represent a local view of the image dataset, wherein the local view has a greater zoom level than the global view.

In some embodiments, following display of the second image rendering, the method may comprise receiving from the user input device of the user interface 52 a second user input command, the same as or different to the first user input command, and wherein, responsive to said receipt, the method comprises switching the display device to display of the first image rendering. In other words toggling between two image renderings may be enabled.

With regards to the user input command which may be received, in some embodiments, the received user input command may be indicative of a location on the first image rendering being displayed. In some cases, the AI classification model 60 may be configured to output anatomical classification data pertaining to a plurality of segmented image regions within the image dataset. The selecting or configuring the system operation may comprise selecting or configuring the system operation based on anatomical or pathology classification in the output classification data assigned to the image region containing the user-indicated location on the image. For example, the user indicates (e.g. clicks or double clicks or any other command or gesture) a particular part of the anatomy depicted in the first image rendering, and the system operation is selected based on which part of the anatomy was selected. For example, a second image rendering representing a zoomed view of the selected part of the anatomy may be generated, wherein the particular zoom level setting is configured in dependence upon the anatomical classification of the anatomy selected. By way of a further example, the user indicates (e.g. clicks or double clicks or any other command or gesture) a particular pathology depicted in the first image rendering (e.g. a lung nodule), and the system operation is selected based on which pathology was selected. For example, a second image rendering representing a zoomed view of the selected part of the anatomy may be generated, wherein the particular zoom level setting is configured in dependence upon the pathology classification of the pathology.

An example implementation of the method according to a particular set of embodiments will now be described by way of illustration of the above-summarized concept of the invention. It will be appreciated that not all features of this particular set of embodiments are essential to the inventive concept, and are described to aid understanding and to provide an example to illustrate the inventive concepts.

The system may comprise a machine learning model zoo comprising a collection or repository of pre-trained machine learning models. Each may be trained for generating anatomical and/or pathology classification data pertaining to a different respective anatomy area, e.g. rib cage, spine, lungs and so on. The anatomical/pathological classification data may comprise anatomical/pathological segmentation data.

The system may permit selection of a desired reading protocol (e.g., assessment of rib cage, spine, lungs, etc.). Based on this, the corresponding machine learning model may be selected from the model zoo. The selected model may be a model trained to identify and localize-i.e., segment-the anatomies or pathologies of interest, e.g., ribs, vertebrae, or lungs. Models dedicated to particular anatomies could be selected and/or models dedicated to detecting particular pathologies, e.g. a lung nodule detection module for lung anatomy, or a rib fracture detection module for thoracic anatomy.

As the anatomical machine learning models are not directly used for diagnostic purposes, e.g., directly delineating fractures, these models may be comparatively fast and lightweight algorithms.

The information obtained from application of the selected model can be used to infer the anatomical structure or pathology clicked on by the user and to thereby select the correct zoom level from a database, e.g. comprised by a command translator, in the next step.

The assessment of different pathologies and different anatomical structures may require different zoom levels and/or viewport (display device) configurations. Therefore, a zoom-level database may be provided which contains gold-standard settings based on the selected reading protocol and inferred anatomical structure or pathology from the previous step. The zoom level database may comprise a set of mapping schemes, one for each possible detected anatomical structure (anatomical classification) or detected pathology (pathology classification) each mapping scheme defining the gold standard zoom settings, and user input commands to be associated with each one. Presenting a given view setting comprises rendering a further image for display corresponding to that view setting.

A different set of mapping schemes may be included for each possible reading protocol. In some embodiments, the system may permit the settings from the database or mapping scheme to be superimposed by personal preference settings input by the user, e.g. an operating physician. A default zoom setting may be selected if the anatomical or pathological classification was not conclusive in identifying the target anatomy or pathology.

A user interaction model may be implemented on the user interface which permits a user to initiate the smart zooming feature by simple gesture. One non-limiting example is a double-click or double-tap on a touch screen. Other non-limiting examples include for instance single tapping with two or more fingers, or a pinch gesture. After providing an input indicating an location on the image, e.g., indicating a rib or a lung nodule, a parallel ray may be projected through the current viewport and the image data. The closest point of intersection of this selection ray with the relevant segmented anatomical/pathological structure, e.g., the rib cage, is computed. Next, a database lookup of the predefined zoom level for this anatomy/pathology may be queried. For example, this may be performed by a command translator 58 module. This information is passed to an image rendering module and a new image is rendered with the desired zoom level. Optionally, the initial zoom level prior to the zoom may be saved and can be returned to by repeating the zoom gesture, even if the viewport has been adjusted in the zoomed-in setting. Optionally, if a specific, highly standardized reading protocol is selected, the focus of the viewport can also jump to the next position instead, e.g., to the next vertebra if the entire spine is to be read.

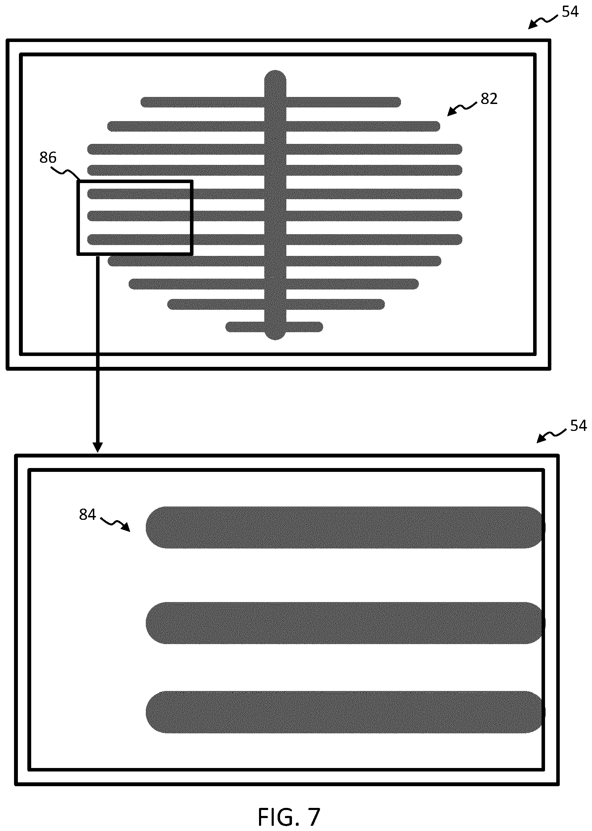

Fig. 7 schematically illustrates an example implementation in accordance with the above outlined example set of embodiments. The first image rendering 82 is first displayed on the display device 54 of the user interface. In this example, the first image rendering is a global view of a CT scan of the ribs of a user. The user clicks on a rib located in the indicated sub-region 86. Responsive to this, the system generates a second image rendering 84 representing the indicated sub-region 86 of the first image rendering 82 at a zoom level determined based on an anatomical classification of the indicated subregion 86. The anatomical classification is determined based on application of the AI anatomical classification model 60 to the underlying image data which yields anatomical segmentation information.

A similar process could be performed in the case of a pathology depicted in the image. For example, the user clicks on a rib fracture depicted in the image. Responsive to this, the system generates a second image rendering representing a sub-region of the first image rendering which contains the depicted pathology at a zoom level determined based on a pathology classification of the indicated pathology. The pathology classification is determined based on application of an AI pathology classification model to the underlying image data which yields pathology detection and/or segmentation information.

The various discussed modules of the system may be software modules or hardware modules. Although these are schematically illustrating as being separate elements, the functionality performed by the different modules could be performed by a common set of one or more processors. The system may be implemented on a local hardware or by a distributed hardware. A cloud computing implementation is also possible.

Embodiments of this invention can be applied in any situation in which medical images are visualized, viewed, assessed and/or read. Besides classical reading rooms and workstations with human-machine interfaces in the defined clinical setting, this includes edge reading of medical images at the point-of-care. In the latter situation, medical experts are often only equipped with smartphones and tablet computers that complicate the navigation in medical image data. Here, embodiments of the invention may be most beneficial and be implemented using familiar input gestures utilized by mobile computing devices with touch-screen interfaces.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) of configuring user input of a medical image visualization system, comprising:
displaying (18) on a display device of a user interface a first image rendering of a medical image dataset;
receiving (22) from a user input device of the user interface a user input command;
applying (20) to the medical image dataset an AI classification model (60) configured to output classification data pertaining to at least a region of the image data;
performing (26) a system operation on the visualization system responsive to the user input command;
wherein the method comprises selecting or configuring (24) the system operation which is performed responsive to the received user input command in dependence upon the classification data, based on a pre-defined command translation protocol.

2. The method of claim 1, wherein:
the AI classification model is an AI anatomical classification model configured to output anatomical classification data pertaining to at least a region of the image data; or
an AI pathology classification model configured to output pathological classification data pertaining to at least a region of the image data.

3. The method of claim 1 or 2, wherein the received user input command is indicative of a location on the first image rendering being displayed.

4. The method of claim 3, wherein
the AI classification model is configured to output classification data pertaining to a plurality of segmented image regions within the image dataset; and
the selecting or configuring the system operation comprises selecting or configuring the system operation based on the classification in the output classification data assigned to the image region containing the user-indicated location on the image.

5. The method of any preceding claim, wherein the system operation comprises a processing operation applied to the image dataset, and optionally wherein the system operation comprises an image rendering operation applied to the image dataset.

6. The method of claim 5, wherein the system operation comprises an image rendering operation applied to the image dataset to generate a second image rendering of the image dataset and outputting the second image rendering to the display device for display.

7. The method of claim 6,
wherein the adjusting the system operation comprises adjusting rendering parameters of the image rendering operation applied to the image dataset to generate the second image rendering, and
optionally wherein the rendering parameters of the image rendering operation include a zoom setting of the image, for example a zoom level and/or a zoom center point.

8. The method of claim 6 or 7, wherein the first image rendering of the image dataset represents a global view of the image dataset, and wherein the second image rendering of the image dataset represents a local view of the image dataset, wherein the local view has a greater zoom level than the global view.

9. The method of any of claims 6-8, wherein, following display of the second image rendering, the method comprises receiving from the user input device of the user interface a second user input command, the same as or different to the first user input command, and wherein, responsive to said receipt, the method comprises switching the display device to display of the first image rendering.

10. The method of any preceding claim,
wherein the user interface comprises a user input mechanism comprising the user input device and a command translator, and wherein the performing the system operation responsive to the user input command comprises the command translator translating the user input command to an assigned system operation command, and
optionally wherein the selecting or configuring the system operation which is performed responsive to the received user input command comprises selecting or configuring the system operation command assigned to the user input command.

11. The method of claim 10, wherein the command translator is configured to translate any of a defined set of possible user input commands to respective assigned system operation commands, and wherein the assignments of the system operation commands to the user input commands is defined by a mapping schema comprised by the command translator, and wherein the mapping schema is adjustable to vary the system operation commands assigned to the user input commands.

12. The method of claim 11,
wherein, the method comprises adjusting the mapping schema to select or configure the respective system operation commands assigned to each of said set of user input commands in dependence upon the output classification data; and
optionally wherein the pre-defined command translation protocol defines how the mapping schema is to be adjusted in dependence upon the classification data.

13. A computer program product comprising computer program code configured, when run on a processor, to cause the processor to perform the method of any of claims 1-12.

14. A processing device (32) for a medical image visualization system, comprising one or more processors (36), configured to:
control a display device (54) of a user interface (52) to display a first image rendering of a medical image dataset;
receive from a user input device of the user interface a user input command;
apply to the medical image dataset an AI classification model (60) configured to output classification data pertaining to at least a region of the image data;
select or configure a system operation which is performed responsive to the received user input command in dependence upon the classification data, based on a pre-defined command translation protocol; and
perform the system operation responsive to the user input command.

15. An image visualization system (30) comprising:
a user interface (52) comprising: a display device and a user input device; and
a processing device (32) in accordance with claim 14, operatively coupled to the user interface; and
optionally wherein the user input device comprises:
a user command detector (56) adapted to detect user commands and generate command signals indicative thereof; and
a command translator (58) adapted to translate the user command signals to system command signals indicative of a system operation to be performed responsive to the user command signal, based on the pre-defined command translation protocol.
